# EUROPEAN PATENT APPLICATION

(11) **EP 4 691 244 A1**
(43) Date of publication of application: **11.02.2026**
(21) Application number: 24780627.6
(22) Date of filing: 28.03.2024
(51) Int. Cl.: A01N 63/40, A01P 3/00, C12N 7/00

(54) **PLANT DISEASE CONTROL COMPOSITION, PLANT DISEASE CONTROL METHOD, AND USE OF ORGANIC SILICONE-BASED SURFACTANT**

(30) Priority: 30.03.2023 JP 2023054722
(71) Applicant: Kaneka Corporation, Osaka-shi, Osaka 530-8288 (JP); Mitsui & Co., Ltd., Chiyoda-Ku Tokyo 100-8631 (JP)
(72) Inventor: DOJUN, Nobuhiko, Iwata-shi, Shizuoka 438-0802 (JP); YOSHIDA, Shinichi, Iwata-shi, Shizuoka 438-0802 (JP); KUDO, Hitoshi, Tokyo 100-8631 (JP)
(74) Representative: Hoffmann Eitle
(86) International application number: PCT/JP2024/012616
(87) International publication number: WO 2024/204527

(57) **Abstract**

An aim of the present invention is to provide a composition for plant disease control containing a bacteriophage, wherein the composition for plant disease control is inhibited from decreasing in the bacteriolytic activity or maintains the bacteriolytic activity, and has excellent persistence characteristics. One aspect of the present invention is a composition for plant disease control, comprising: a bacteriophage with bacteriolytic activity against bacteria; and an organic silicone-based surfactant.

## Description

### Technical Field

The present invention relates to a composition for plant disease control, a method for controlling plant disease, and use of an organic silicone-based surfactant.

### Background Art

To control harmful bacteria causative of various plant diseases in crops, an agricultural chemical compound, such as a copper agent or an antibiotic, has been used. However, there are many problems, such as a drug effect, drug poisoning, and as well as possible causes for the disruption of a bacterial flora balance. Hence, recent interest has focused on a method using a bacteriophage as a new control method. A bacteriophage (herein also referred to as a "phage") is a generic term for viruses that infect only bacteria. Many phages, also called bacteriolytic phages, specifically attach to target host bacteria, then inject their own DNA, and self-amplify by utilizing the translational mechanism of the bacteria. Furthermore, the bacteria are bacteriolyzed, and consequently, the amplified phages are propagated, and an infection into new target bacteria is repeated. A biopesticide for controlling harmful bacteria causative of a plant disease by utilizing such bacteriolytic activity of a bacteriophage has been developed and studied.

For example, Patent Literature 1 discloses a composition for plant disease biocontrol formulated for delivery to a plant, wherein the composition comprises at least one kind of carrier and at least one bacteriophage selected from the group consisting of the Xfas100 phage type and the Xfas300 phage type, wherein the bacteriophage is virulent to *Xylella fastidiosa* and *Xanthomonas* species (Claim 12). Additionally, Patent Literature 1 states that such a composition can be applied by spraying, misting, or scattering on the plant, and that the carrier may comprise a diluent, a surfactant, and/or a buffer.

### Citation List

### [Patent Literature]

[Patent Literature 1] JP2015-534983A

### Summary of Invention

### Technical Problem

Unlike a conventional copper agent and/or antibiotic, a bacteriophage, which is one species of virus, is a natural product, and the manifestation of its drug poisoning has hitherto not been reported. Additionally, a phage has a comparatively high specificity to a host, and thus, only targets bacteria of a specific genus or species, having an extremely limited influence on a bacterial flora balance. Additionally, a phage is harmless to not only an animal such as a human but also to a plant, and is very safe. Hence, a bacteriophage is a control means that has been attracting much attention in recent years.

A method for applying the above-described biopesticide to a target plant to be controlled is, for example, a method for application by dispersing or the like on a leaf face or the like of a plant, as described in Patent Literature 1. In some of the cases where a conventional agricultural chemical compound, such as a copper agent and/or an antibiotic, is used, a sticking agent is added to the agricultural chemical composition to enhance the residual amount on the leaf face and/or stem, and thereby ensure the drug efficacy. As such a sticking agent, any of various surfactants can be used for an agricultural chemical compound. However, the characteristics, properties, dynamics, and the like of a bacteriophage are obviously different from the characteristics, properties, dynamics, and the like of an agricultural chemical compound, such as a copper agent and/or an antibiotic. Thus, applying the sticking agent usable for an agricultural chemical compound directly for a biopesticide containing a bacteriophage is not necessarily possible for the purpose of enhancing the residual amount of a bacteriophage in a plant. Specifically, a bacteriophage is one species of virus, and thus, mixing a bacteriophage with a surfactant as a sticking agent can cause a problem, such as, being unable to obtain desired persistence characteristics and/or desired bacteriolytic activity (efficacy).

In view of this, an object of the present invention is to provide a composition for plant disease control comprising a bacteriophage, wherein the composition for plant disease control is inhibited from decreasing in the bacteriolytic activity or maintains the bacteriolytic activity, and has excellent persistence characteristics.

### Solution to Problem

To solve the above-described problems, the present inventors have made studies vigorously, and have reached the present invention through the discovery that using an organic silicone-based surfactant enables a composition for plant disease control to be provided, wherein the composition is inhibited from decreasing in the bacteriolytic activity or maintains the bacteriolytic activity, and has excellent persistence characteristics.

In view of this, examples of aspects according to the present invention are as follows.
(1) A composition for plant disease control, comprising: a bacteriophage with bacteriolytic activity against bacteria; and an organic silicone-based surfactant.
(2) The composition for plant disease control according to (1), wherein the organic silicone-based surfactant is a silicone oil having a polyether group(s).
(3) The composition for plant disease control according to (1) or (2), wherein the organic silicone-based surfactant is at least one selected from the group consisting of trisiloxaneethoxylate, polyoxyethylene methylpolysiloxane, polyoxyalkylene methylpolysiloxane, and a polyether polymethylsiloxane copolymer.
(4) The composition for plant disease control according to any one of (1) to (3), wherein the organic silicone-based surfactant is trisiloxaneethoxylate.
(5) The composition for plant disease control according to any one of (1) to (4), wherein the organic silicone-based surfactant is polyalkylene-oxide-modified heptamethyltrisiloxane.
(6) The composition for plant disease control according to any one of (1) to (5), further comprising an agriculturally acceptable carrier and/or an agriculturally acceptable solvent.
(7) A method for controlling plant disease, comprising a step of contacting the composition for plant disease control according to any one of (1) to (6) to a target plant.
(8) A method for controlling plant disease, comprising: a step of preparing a composition for plant disease control by mixing a bacteriophage with bacteriolytic activity against bacteria with an organic silicone-based surfactant; and a step of contacting the composition for plant disease control to a target plant to be controlled.
(9) The method for controlling plant disease according to (8), wherein the organic silicone-based surfactant is a silicone oil having a polyether group(s).
(10) The method for controlling plant disease according to (8) or (9), wherein the organic silicone-based surfactant is at least one selected from the group consisting of trisiloxaneethoxylate, polyoxyethylene methylpolysiloxane, polyoxyalkylene methylpolysiloxane, and a polyether polymethylsiloxane copolymer.
(11) The method for controlling plant disease according to any one of (8) to (10), wherein the organic silicone-based surfactant is trisiloxaneethoxylate.
(12) The method for controlling plant disease according to any one of (8) to (11), wherein the organic silicone-based surfactant is polyalkylene-oxide-modified heptamethyltrisiloxane.
(13) The method for controlling plant disease according to any one of (8) to (12), wherein the step of preparing comprises preparing a composition for plant disease control by further mixing an agriculturally acceptable carrier and/or an agriculturally acceptable solvent in addition to the bacteriophage and the organic silicone-based surfactant.
(14) Use of an organic silicone-based surfactant in a composition for plant disease control containing a bacteriophage with bacteriolytic activity against bacteria, wherein the use is for enhancing the residual amount of the composition for plant disease control in a target plant.
(15) Use of an organic silicone-based surfactant in the production of a composition for plant disease control containing a bacteriophage with bacteriolytic activity against bacteria.

The present specification encompasses the disclosure of Japanese Patent Application Nos. 2023-054722 that serves as a basis for the priority of the present application.

### Advantageous Effects of Invention

One aspect of the present invention can provide a composition for plant disease control comprising a bacteriophage, wherein the composition for plant disease control is inhibited from decreasing in the bacteriolytic activity or maintains the bacteriolytic activity, and has excellent persistence characteristics.

### Brief Description of Drawings

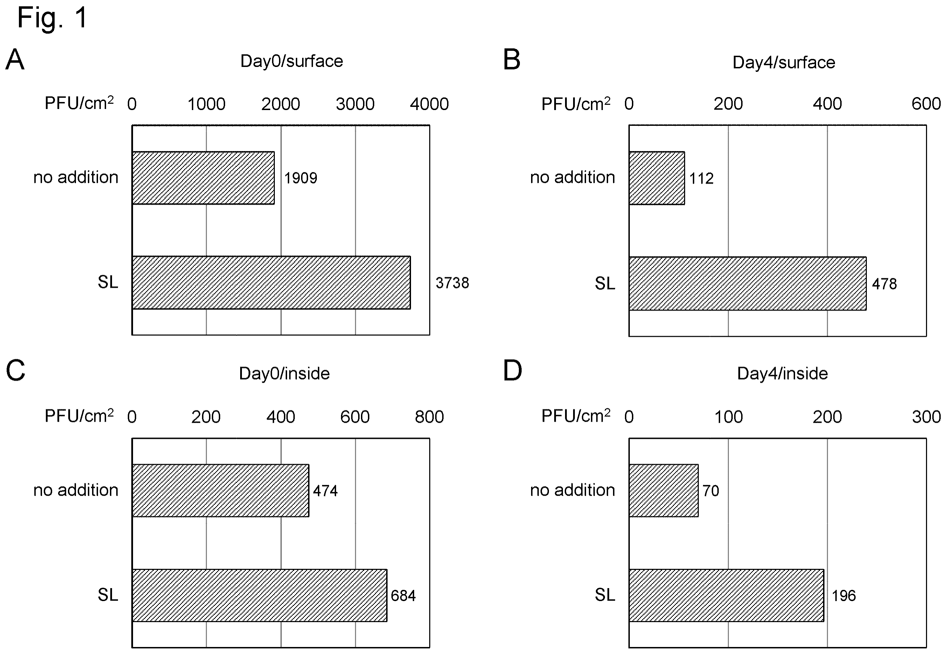

[Figure 1] Figure 1 relates to a test example (Comparative Example 2) having no sticking agent added therein and a test example (Example 2) having an organic silicone-based sticking agent added therein. Figure 1A graphs the residual amount (PFU/cm²) of a phage on the leaf surface of the day 0 sample; Figure 1B graphs the residual amount (PFU/cm²) of a phage on the leaf surface of the day 4 sample; Figure 1C graphs the residual amount (PFU/cm²) of a phage inside the leaf of the day 0 sample; and Figure 1D graphs the residual amount (PFU/cm²) of a phage inside the leaf of the day 4 sample.

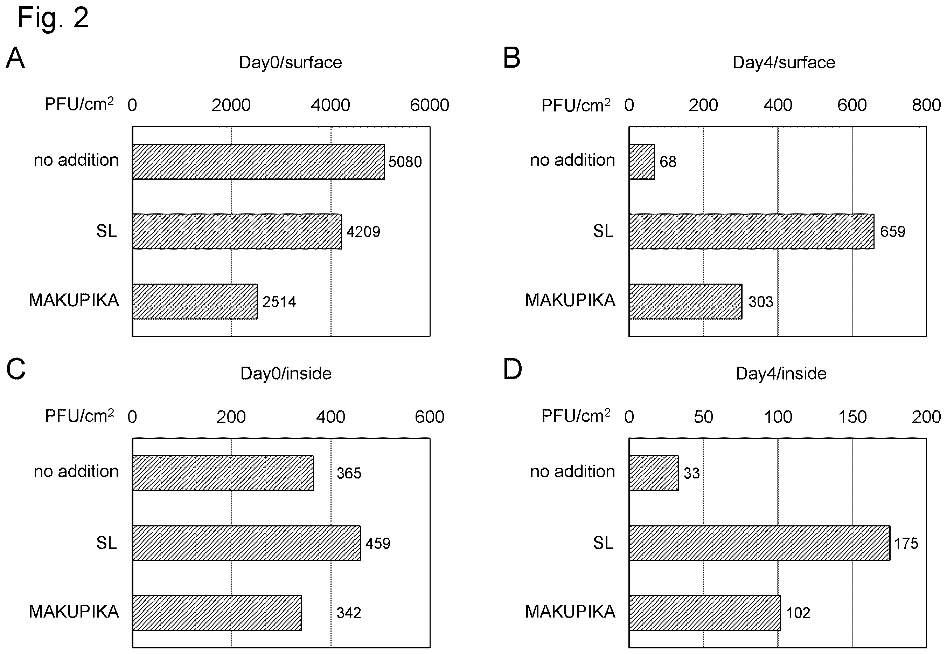

[Figure 2] Figure 2 relates to test examples including use of a 4th phage, as follows: a test example (Comparative Example 3-1) having no sticking agent added therein; a test example (Example 3-1) having an organic silicone-based sticking agent (Silwet L-77 (hereinafter referred to as SL)) added therein; or a test example (Example 3-2) having an organic silicone-based sticking agent (MAKUPIKA) added therein. Figure 2A graphs the residual amount (PFU/cm²) of a phage on the leaf surface of the day 0 sample; Figure 2B graphs the residual amount (PFU/cm²) of a phage on the leaf surface of the day 4 sample; Figure 2C graphs the residual amount (PFU/cm²) of a phage inside the leaf of the day 0 sample; and Figure 2D graphs the residual amount (PFU/cm²) of a phage inside the leaf of the day 4 sample.

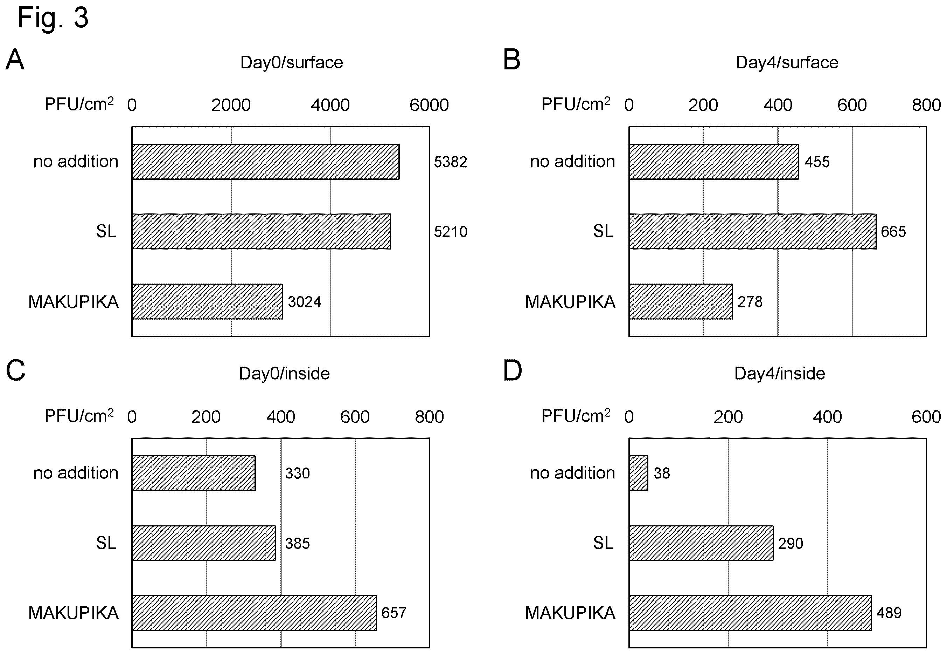

[Figure 3] Figure 3 relates to test examples including use of a 5th phage, as follows: a test example (Comparative Example 3-2) having no sticking agent added therein, a test example (Example 3-3) having an organic silicone-based sticking agent (SL) added therein, or a test example (Example 3-4) having an organic silicone-based sticking agent (MAKUPIKA) added therein. Figure 3A graphs the residual amount (PFU/cm²) of a phage on the leaf surface of the day 0 sample; Figure 3B graphs the residual amount (PFU/cm²) of a phage on the leaf surface of the day 4 sample; Figure 3C graphs the residual amount (PFU/cm²) of a phage inside the leaf of the day 0 sample; and Figure 3D graphs the residual amount (PFU/cm²) of a phage inside the leaf of the day 4 sample.

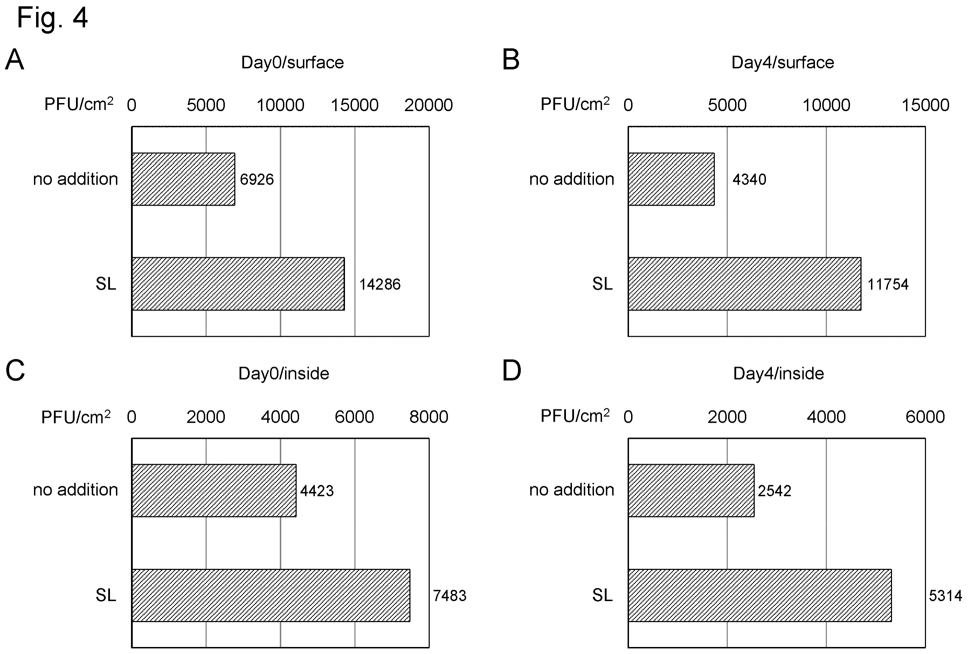

[Figure 4] Figure 4 relates to test examples including use of an excised broccoli leaf, as follows: a test example (Comparative Example 4-1) having no sticking agent added therein and a test example (Example 4-1) having an organic silicone-based sticking agent (SL) added therein. Figure 4A graphs the residual amount (PFU/cm²) of a phage on the leaf surface of the day 0 sample; Figure 4B graphs the residual amount (PFU/cm²) of a phage on the leaf surface of the day 4 sample; Figure 4C graphs the residual amount (PFU/cm²) of a phage inside the leaf of the day 0 sample; and Figure 4D graphs the residual amount (PFU/cm²) of a phage inside the leaf of the day 4 sample.

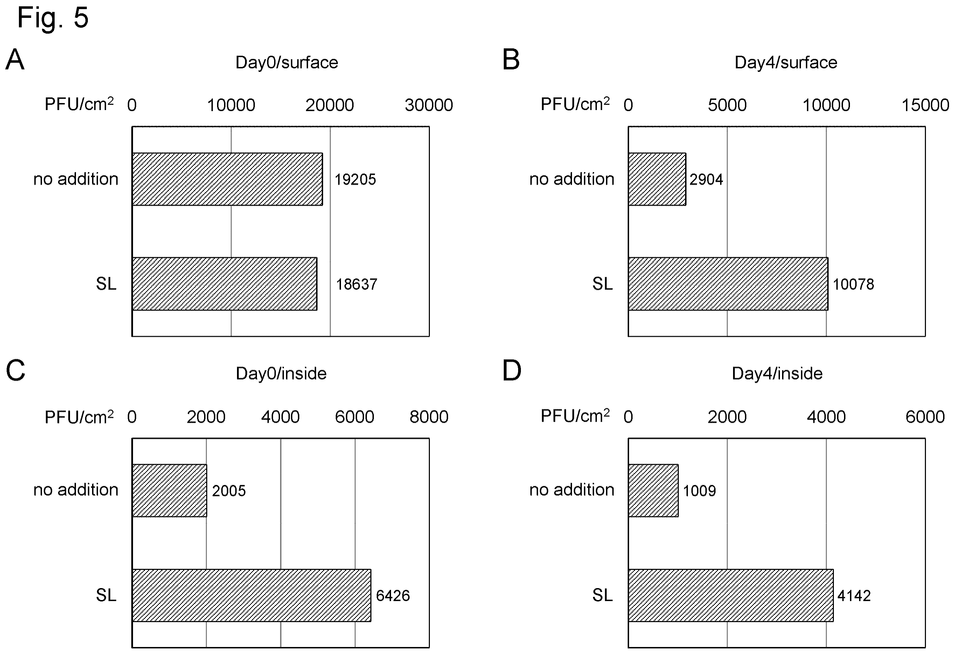

[Figure 5] Figure 5 relates to test examples including use of an excised tomato leaf, as follows: a test example (Comparative Example 4-2) having no sticking agent added therein and a test example (Example 4-2) having an organic silicone-based sticking agent (SL) added therein. Figure 5A graphs the residual amount (PFU/cm²) of a phage on the leaf surface of the day 0 sample; Figure 5B graphs the residual amount (PFU/cm²) of a phage on the leaf surface of the day 4 sample; Figure 5C graphs the residual amount (PFU/cm²) of a phage inside the leaf of the day 0 sample; and Figure 5D graphs the residual amount (PFU/cm²) of a phage inside the leaf of the day 4 sample.

### Description of Embodiments

### 1. Definition

Terms used herein are defined below.

A term "plant disease" as used herein is a generic term for diseases that develop in a plant. A known plant disease includes a disease caused by an infectious pathogen such as a virus, bacterium, filamentous fungus, actinomycete, viroid, *Phytoplasma,* nematode, mite, or insect, and a disease caused by a noninfectious pathogen such as the lack or excess of a nutrient or water, drug poisoning, or the like. A plant disease herein refers to a disease whose pathogens are bacteria, i.e., a plant pathogenic bacterium, unless otherwise specified.

A term "control" as used herein refers to prevention or treatment (extermination) (based on the home page of the Japan Crop Protection Association). Accordingly, "plant disease control" as used herein refers to the prevention of a plant disease, particularly against a target bacterium, or to the treatment of a plant disease caused by a target bacterium.

A term "target plant" as used herein refers to a plant to which the composition for plant disease control according to the present invention is to be applied. This plant is a plant that has a specific plant disease caused by the infection of the target bacteria, or a plant that has the possibility of being infected by the target bacteria.

### 2. Composition for Plant Disease Control

### 2-1. Overview

One aspect of the present invention is a composition for plant disease control. The composition for plant disease control according to the present invention comprises: a bacteriophage with bacteriolytic activity against bacteria; and an organic silicone-based surfactant.

The composition for plant disease control according to the present invention is inhibited from decreasing in the bacteriolytic activity or maintains the bacteriolytic activity, and has excellent persistence characteristics. The composition exhibits bacteriolytic activity against a target bacterium as a potential pathogenic bacterium of a plant disease, and also has excellent persistence characteristics in a plant. Additionally, using the composition for plant disease control according to the present invention for plant disease control can provide sustainable plant protectives (agricultural chemicals) against a bacterial plant disease, wherein the protectives are safe for the human body, free from drug poisoning to the environment, and capable of preventing or treating a plant disease of interest specifically. The phrase "inhibited form decreasing in the bacteriolytic activity or maintains the bacteriolytic activity" means that the bacteriolytic activity of the composition for plant disease control according to the present invention corresponds to, for example, 85% or more, preferably 90% or more, 95% or more, or 100% or more, of the bacteriolytic activity of a composition for plant disease control having the same composition except for containing no organic silicone-based surfactant. The bacteriolytic activity of the composition can be expressed as a titer based on the plaque forming unit (PFU) in a plaque assay method, and, for example, can be measured using a plaque assay method after the composition is left to stand in a thermostatic chamber at 25°C overnight (approximately 16 hours).

The term "composition for plant disease control" as used herein refers to a composition used for plant disease control.

### 2-2. Constitution

### 2-2-1. Component

The composition for plant disease control according to the present invention comprises, as an essential component, a bacteriophage that, in the form of an active ingredient, exhibits bacteriolytic activity against bacteria. Additionally, the composition for plant disease control according to the present invention also comprises an organic silicone-based surfactant as an essential component. Using an organic silicone-based surfactant enables enhancement of the residual amount of a bacteriophage as an active ingredient after application of the bacteriophage to a plant. Additionally, the composition for plant disease control according to the present invention may comprise an agriculturally acceptable carrier and/or an agriculturally acceptable solvent. The agriculturally acceptable carrier and/or the agriculturally acceptable solvent preferably do/does not inhibit or suppress the bacteriolytic activity against a target bacterium for the phage. Furthermore, the composition for plant disease control according to the present invention may comprise another active ingredient, if desired. Below, each component will be described specifically.

### (1) Bacteriophage

The composition for plant disease control according to the present invention comprises, as an essential active ingredient, a bacteriophage with bacteriolytic activity against bacteria. Because this active ingredient bacteriolyzes the target bacteria of the present invention, the composition for plant disease control can prevent or treat a plant disease caused by the target bacteria. The bacteriophage may be used alone, or two or more species thereof may be used in combination.

In the present invention, a bacteriophage is not particularly limited as long as it has bacteriolytic activity against a target bacterium. Many bacteriophages are specific to a specific genus, species, or strain of bacteria. The term "bacteriophage" has the same meaning as the term "phage".

The bacteriophage is not particularly limited, and is, for example, a bacteriophage that belongs to any of the following virus families: Myoviridae, Siphoviridae, Podoviridae, Autographiviridae, Ackermannviridae, Corticoviridae, Cystoviridae, Inoviridae, Leviviridae, Microviridae, or Tectiviridae. The bacteriophage can be a bacteriolytic bacteriophage. The bacteriolytic bacteriophage does not enter a lysogenic pathway, and traces a bacteriolytic pathway through completion of a bacteriolytic cycle.

Additionally, the bacteriophage in the present invention may be derived from the Caudovirales phages. Caudovirales is the order of bacteriophages having a double-strand DNA (dsDNA) genome, and having a tail. Each virion of the Caudovirales order has a virion genome and an icosahedral head containing a flexible tail. The Caudovirales order includes, for example, the following bacteriophage families: Myoviridae (a long contractile tail), Siphoviridae (a long noncontractile tail), Podoviridae (a short noncontractile tail), Autographiviridae, and Ackermannviridae.

When the composition is used for plant disease control, the amount of the active ingredient comprised per unit amount in the composition for plant disease control depends on various conditions, such as the dosage form, the kind of plant pathogenic bacteria, the kind of a target plant, the site of application, and the method of application. The amount to be comprised is preferably sufficient for the phage, as an active ingredient, to contact and infect plant pathogenic bacteria that have infected a target plant. Accordingly, it can be determined within the scope of common technical knowledge in the art, considering the conditions such that the bacteriolytic agent comprised in the composition for plant disease control according to the present invention is in an effective amount for target bacteria after application.

The concentration of the bacteriophage in the composition for plant disease control according to the present invention is not particularly limited, and can be suitably set in accordance with the kind or degree of a disease as a target for application, the method for application, or the dosage form. The concentration of the bacteriophage is, for example, preferably 1 × 10³ PFU/mL or more, preferably 1 × 10⁴ PFU/mL or more, preferably 1 × 10⁵ PFU/mL or more, preferably 1 × 10⁶ PFU/mL or more, or preferably 1 × 10⁷ PFU/mL or more. Additionally, the concentration of the bacteriophage is, for example, preferably 10¹⁵ PFU/mL or less, preferably 10¹⁴ PFU/mL or less, or preferably 10¹³ PFU/mL or less. An upper limit value and a lower limit value from these value ranges can be arbitrarily combined to prescribe a preferred range.

The composition for plant disease control according to the present invention may comprise, as an active ingredient(s), one or more species of phages in combination. For example, even if target bacteria are the same between phages, the phages, if capable of recognizing different cell surface receptors, can be expected to allow a synergistic effect and/or supportive effect of the bacteriolytic activity, depending on the combination. Additionally, even though target bacteria are different, the phages can achieve bacteriolytic activity against the different bacteria, and can be expected to have control activity against pathogens in a wider range.

### (2) Organic silicone-based surfactant

The composition for plant disease control according to the present invention comprises an organic silicone-based surfactant as an essential component. The organic silicone-based surfactant may be used alone, or two or more kinds thereof may be used in combination. For example, the reason why containing an organic silicone-based surfactant enables a bacteriophage to be inhibited from decreasing in, or maintain, the bacteriolytic activity, and simultaneously enhance the persistence characteristics, although the reason is by inference, is as follows.

The reason why bacteriolytic activity is inhibited from decreasing or why bacteriolytic activity is maintained is considered to be that an organic silicone-based surfactant allows a bacteriophage to function without being modified. The capsid of a bacteriophage and the tail fiber important for contact with a target host are composed of protein. Depending on the kind of a sticking agent to be mixed in, the bacteriolytic activity of a bacteriophage can be decreased remarkably. This is considered to be because such a sticking agent that remarkably decreases the bacteriolytic activity of a bacteriophage modifies the phage to thereby make the phage unable to exert the bacteriolytic activity against a disease bacterium. On the other hand, an organic silicone-based surfactant conceivably does not modify a bacteriophage, and thus, allows the bacteriophage to be inhibited from decreasing in, or maintain, the bacteriolytic activity. Furthermore, the residual amount of the bacteriophage is increased conceivably because the surfactant action of organic silicone-based surfactant makes it easy for a bacteriophage to have access to a plant cell of a plant leaf.

An organic silicone-based surfactant (also referred to as an organic silicone-based sticking agent) mainly refers to a silicone oil having hydrophilicity given by introduction of the organic functional group, such as polyether group, and, for example, refers to a silicone oil having a polyether group(s). As an organic group to be introduced into the organic silicone-based surfactant, various organic groups other than the above-described polyether group are known, and can be used as long as they serve an object of the present disclosure. Examples of organic silicone-based surfactants having a polyether group(s) introduced thereinto include a compound represented by the following structural formula (I): 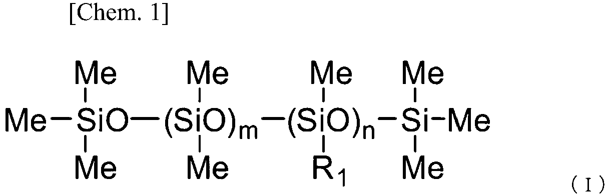 [wherein, in the formula (I),
R₁ is an organic group represented by the following general formula (II):

   -R₂-O-(C₂H₄O)ₓ-(C₃H₆O)_{y}-R₃ (II)

   (wherein, in the formula (II), R₂ is an unsubstituted or substituted C₂₋₆ alkylene group; R₃ is a hydrogen atom, an unsubstituted or substituted C₁₋₆ alkyl group, or an acetyl group (-COCH₃); x is an integer of 0 to 15; and y is an integer of 0 to 10);
Me is a methyl group;
m is an integer of 0 to 10; and
n is an integer of 1 to 10].

Desirably, in the structural formula (I),
R₁ is an organic group represented by the following general formula (III):

   -R₂-O-(C₂H₄O)ₓ-R₃ (III)

   (wherein, in the formula, R₂ is a propylene group; R₃ is a hydrogen atom or a methyl group; and x is an integer of 0 to 15);
Me is a methyl group;
m is an integer of 0 to 3; and
n is 1.

The organic silicone-based surfactant is preferably at least one selected from the group consisting of trisiloxaneethoxylate, polyoxyethylene methylpolysiloxane, polyoxyalkylene methylpolysiloxane, and a polyether polymethylsiloxane copolymer. Among these, trisiloxaneethoxylate, polyoxyethylene methylpolysiloxane, or trisiloxaneethoxylate is preferable, and trisiloxaneethoxylate is more preferable.

Specific examples of organic silicone-based surfactants include the following (tradenames). Examples of trisiloxaneethoxylate include Silwet L-77, Silwet 408, or Silwet 440 (registered trademarks; manufactured by Momentive Performance Materials, Inc.). Examples of polyoxyethylene methylpolysiloxane include MAKUPIKA (registered trademark; manufactured by Ishihara Sangyo Kaisha, Ltd.). Examples of polyoxyalkylene methylpolysiloxane include KF-640 (manufactured by Shin-Etsu Chemical Co., Ltd.). Examples of the polyether polymethylsiloxane copolymer include BREAK-THRU (registered trademark; manufactured by Evonik Goldschmidt Chemical Corporation) or BREAK-THRU (registered trademark; manufactured by Sankei Chemical Co., Ltd.). Additionally, some commercially available organic silicone-based surfactants further contain another component.

The trisiloxaneethoxylate is more preferably, for example, polyalkylene-oxide-modified heptamethyltrisiloxane "Silwet L-77".

The amount of an organic silicone-based surfactant contained in the composition is not particularly limited, and can be suitably selected, considering various conditions, such as the species of plant pathogenic bacteria, the species of a target plant, the site of application, and the method for application. For example, the commercially available product Silwet L-77 can be added to the composition for plant disease control in such a manner that the amount of the product contained in the composition is diluted, for example, 500-fold to 5000-fold (preferably 1000-fold to 3000-fold) with respect to the stock solution.

### (3) Agriculturally acceptable carrier and/or agriculturally acceptable solvent

The composition for plant disease control according to the present invention may comprise an agriculturally acceptable carrier and/or an agriculturally acceptable solvent.

An agriculturally acceptable carrier refers to a substance that facilitates the application of a composition, can maintain the survivability and infectiousness of a phage as an active ingredient, and/or can regulate the rate of action. The agriculturally acceptable carrier may be a substance that has no or an extremely small harmful influence on an environment, such as the soil and the water quality, when applied outdoor, and that further has no or an extremely low harmfulness to an animal, particularly a human.

Specific examples of agriculturally acceptable carriers include: surfactants other than an organic silicone-based surfactant; protective agents; excipients and the like. Additionally, if desired, a moisturizer, emulsifying agent, pH buffering agent, and the like may be utilized in small amounts. The carrier may be blended in advance, or may be blended immediately before application.

The surfactant has an effect on enhancing the physicochemical properties of the composition against parts of a plant, in which the properties include hygroexpansivity (wetting capability), emulsifying capability, dispersibility, permeating capability, adhesiveness, defoaming capability, and spreading capability. The surfactant may be utilized as a main component of an agricultural adjuvant called a sticking agent. The sticking agent is, for example, a nonionic surfactant, anionic surfactant, cationic surfactant, or a combination thereof. More specific examples include polyoxyethylenealkyl ether-based compounds, polyoxyethylene fatty acid ester-based compounds, lignin sulfonic acid salt-based compounds, naphthylmethanesulfonic acid salt-based compounds, alkylsulfosuccinic acid salt-based compounds, or tetraalkylammonium salt-based compounds. However, a surfactant (other than an organic silicone-based surfactant) can influence the bacteriolytic activity of a phage. Thus, adding the surfactant in such an amount as to practically impair the effect of the present invention is desirably avoided. For example, the amount of the surfactant (other than an organic silicone-based surfactant) contained in the composition for plant disease control is preferably 10 mass% or less, preferably 5 mass% or less, preferably 3 mass% or less, preferably 1 mass% or less, preferably 0.5 mass% or less, preferably 0.1 mass% or less, preferably 0.05 mass% or less, preferably 0.01 mass% or less, or preferably 0.001 mass% or less.

A protective agent is expected to have an effect such as decreasing damage for a phage due to ultraviolet rays. Examples include skim milks, caseins, gelatins or the like.

Examples of excipients include glucose, lactose, sucrose, gelatin, starch, malt, flours or the like.

Specific examples of agriculturally acceptable solvents include water (including an aqueous solution), buffers, liquid culture media, or the like. The solvent is, preferably, an aseptic liquid.

### (4) Another active ingredient

The composition for plant disease control according to the present invention may comprise, in addition to the above-described bacteriophage, one or more other active ingredients having different pharmacologic actions. Examples of the other active ingredients include insecticides, nematicides, miticides, herbicides, plant growth promoters, antibiotics, biological agricultural chemicals and the like.

### 2-2-2. Dosage form

The dosage form of the composition for plant disease control according to the present invention may be any dosage form, as long as it is possible that the fixability to a site of infection of target bacteria on a target plant or a target plant, and/or the susceptible to infection of a phage as an active ingredient against target bacteria is maintained. The composition for plant disease control may be, for example, a liquid or a water-dispersible agent in a liquid state, obtained by suspending a bacteriophage in a suitable solution. For example, when the site of infection of target bacteria on a target plant is a leaf, flower, fruit, stem, branch, or trunk in the aerial part, a liquid, water-dispersible agent, or gel, which widely spreads in such a site of infection, and has high fixability, is suitable, without limitation.

### 2-3. Method for preparation

The composition for plant disease control according to the present invention can be prepared, for example, by mixing the following: a bacteriophage with bacteriolytic activity against bacteria; and an organic silicone-based surfactant. Additionally, an agriculturally acceptable carrier and/or an agriculturally acceptable solvent may be further mixed in addition to the bacteriophage and the organic silicone-based surfactant to prepare the composition for plant disease control. The bacteriophage may be provided in the form of a liquid or solid bacteriolytic agent. The bacteriolytic agent contains a bacteriophage as an active ingredient, and itself can have bacteriolytic activity. The bacteriolytic agent is mixed with an organic silicone-based surfactant and, if desired, an agriculturally acceptable carrier and/or medium during or immediately before application to a plant, and used to prepare the composition for plant disease control. The bacteriolytic agent may be, for example, a liquid or a water-dispersible agent in a liquid state, obtained by suspending a bacteriophage in a suitable solution, or may be mixed with a carrier and solidified to be formed into a solid-state powder, granule, or gel. The bacteriolytic agent can be provided in a state where a bacteriophage is concentrated. For example, the bacteriolytic agent may be a culture preparation of a bacteriophage used directly or diluted, or may be prepared in the form of a suspension obtained by suspending a bacteriophage in a solvent, such as water or a buffer solution.

### 2-4. Method for application

The method for applying the composition for plant disease control according to the present invention is not particularly limited as long as it enables the composition for plant disease control to come into contact with a target plant to be controlled, and, is for example, a method for direct spraying or scattering on crops, or a method for scattering on soil. Additionally, the composition for plant disease control may be directly dispersed or sprayed, or may be diluted with water or the like, if desired, and applied.

The amount of application of the composition for plant disease control differs depending on the crop as a target for application, target disease, method for application, occurrence tendency, degree of damage, environment conditions, dosage form to be used, or the like, and thus, is desirably adjusted suitably.

### 2-5. Target Plant

A target plant for the composition for plant disease control according to the present invention is not particularly limited to any kind, as long as it is possible to develop a plant disease caused by bacteria. The target plant may be either an angiosperm or a gymnosperm. Furthermore, the plant may be an herbaceous plant or a woody plant. Suitable specific examples of target plants include agriculturally important plants, for example: crop plants such as cereal crops, vegetables, and fruits; and flowers and ornamental plants. Specific examples include monocotyledons, such as Poaceae plants (for example, rice, wheat, barley, corn, sugarcane, *Sorghum,* kaoliangs, and turfgrasses), Musaceae plants (for example, bananas), Amaryllidaceae plants (for example, *Allium,* onions, garlic, and garlic chives), and Liliaceae plants (for example, lilies and tulips). Additional examples include dicotyledons, such as Brassicaceae plants (for example, broccoli, cabbages, radishes, Chinese cabbages, and rapeseeds), Asteraceae plants (for example, lettuces, burdocks, and chrysanthemums), Fabaceae plants (for example, soybeans, peanuts, garden peas, phaseoli, lentils, chickpeas, fava beans, and licorice), Solanaceae plants (for example, tomatoes, eggplants, potatoes, tobaccos, bell peppers, red peppers, and petunia), Rosaceae plants (for example, strawberries, apples, pears, peaches, *Eriobotrya,* almonds, plums, roses, Japanese apricots, and cherries), Cucurbitaceae plants (for example, cucumbers, gourd, pumpkins, melons, and watermelons), Anacardiaceae plants (for example, mangoes, pistachios, and cashew nuts), Lauraceae plants (for example, avocadoes) Rutaceae plants (for example, mandarin oranges, grapefruits, lemons, and *Citrus junos),* Convolvulaceae plants (for example, sweet potatoes), Theaceae plants (for example, *Camellia sinensis),* and Vitaceae plants (for example, grapes).

### 2-6. Target plant disease

A target plant disease to which the composition for plant disease control according to the present invention is to be applied is any plant disease that is caused by bacteria, and is not particularly limited. Merely as one example, the target plant disease is, for example, a plant disease caused by bacteria *of Xanthomonas* genus. Examples include: bacterial spot (bacterial shot hole) observed in peaches and the like; bacterial blight observed in walnuts and the like; bacterial pustule observed in soybeans and the like; angular leaf spot observed in strawberries and the like; bacterial blight observed in tomatoes, bell peppers, lettuces, and the like; black rot observed in cabbages, Chinese cabbages, broccoli, and the like; bacterial canker observed in oranges, grapefruit, and the like; angular leaf spot observed in cotton and the like; and leaf blight observed in rice and the like. Other examples include: fire blight caused by bacteria of *Erwinia* genus and observed in an apple and the like; bacterial soft rot caused by bacteria of *Pectobacterium* genus and observed in *Allium* and the like; and ear soft rot caused by bacteria of *Burkholderia* genus and observed in corn and the like.

### 2-7. Effects

The composition for plant disease control according to the present invention has excellent bacteriolytic activity and excellent phage residual characteristics after application to a plant, and thus, is useful for preventing and inhibiting a disease due to a target bacterium. Additionally, since a phage is an organism-derived substance, there is no report of the manifestation of drug poisoning, and since a phage has high specificity, its influence on the balance of a bacterial flora is extremely limited.

### 3. Method for Controlling Plant Disease

One aspect of the present invention is a method for controlling plant disease. The method for controlling plant disease according to the present invention is characterized by contacting the composition for plant disease control according to the present invention to a target plant to control a plant disease of the target plant.

That is, one aspect of the present invention is a method for controlling plant disease, comprising a step of contacting the composition for plant disease control according to the present invention to a target plant.

Another aspect of the present invention is a method for controlling plant disease, comprising: a step of preparing a composition for plant disease control by mixing a bacteriophage with bacteriolytic activity against bacteria with an organic silicone-based surfactant; and a step of contacting the composition for plant disease control to a target plant to be controlled.

Herein, the "contact" refers to contact between a composition for plant disease control and a target plant. More specifically, the "contact" refers to contact between a composition for plant disease control and at least a part of a target plant. This contacting step is intended to infect a phage as an active ingredient with a target bacterium, whereby the target bacteria are bacteriolyzed. As a result, an effect on controlling the plant disease caused by target bacteria can be achieved.

The contact is preferably direct contact. The direct contact means that a composition for plant disease control, in liquid form or gel form, is spread, sprayed, scattered, or immersed on a target plant. In this case, the site of a plant as a target of contact is mainly a leaf, flower, fruit, stem, branch, and/or trunk.

### 4. Use of organic silicone-based surfactant

One aspect of the present invention is use of an organic silicone-based surfactant.

That is, one aspect of the present invention is use of an organic silicone-based surfactant in a composition for plant disease control containing a bacteriophage with bacteriolytic activity against bacteria, wherein the use is for enhancing the residual amount of the composition for plant disease control in a target plant.

Another aspect of the present invention is use of an organic silicone-based surfactant in the production of a composition for plant disease control containing a bacteriophage with bacteriolytic activity against bacteria.

### Examples

The present invention will be described in further detail below with reference to Examples. The present invention is not limited to these Examples.

### [Phages used]

In the present test example, the following phages were used.
1st phage (Myoviridae): SEQ ID NO: 13
2nd phage (Autographiviridae): SEQ ID NO: 14
3rd phage (Myoviridae): SEQ ID NO: 15
4th phage (Siphoviridae): SEQ ID NO: 12
5th phage (Siphoviridae): SEQ ID NO: 18
6th phage (Myoviridae): SEQ ID NO: 11
7th phage (Siphoviridae): SEQ ID NO: 17
8th phage (Autographiviridae): SEQ ID NO: 16

### <Example 1 and Comparative Example 1>

### (Aim)

The influence of a mixture of a sticking agent and phage on the bacteriolytic activity is confirmed.

### (Method and Results)

### Preparation of solution of mixture of phage and sticking agent, and evaluation of the activity

Silwet L-77 (manufactured by Momentive Performance Materials, Inc.), which is a nonionic surfactant and also an organic silicone-based surfactant, was added to, and mixed with, an aqueous phage solution containing the 1st phage (Myoviridae), the 2nd phage (Autographiviridae), or the 3rd phage (Myoviridae) to prepare the respective phage compositions. In the preparation of the compositions, the concentration of Silwet L-77 was adjusted so as to be 3000-fold diluted. The phage concentration of the solution prepared is commonly expressed using the titer [PFU/mL] based on the plaque forming unit (PFU) in a plaque assay method. Each phage composition was prepared in such a manner that the titer measured immediately after preparation was 10⁴ PFU/mL. Each phage composition having no sticking agent added therein was also prepared with addition of water so as to have the same degree of titer (10⁴ PFU/mL).

In Comparative Example, DRIVER (registered trademark; 24.0% of polyoxyethylene fatty acid ester; manufactured by Maruwa Biochemical Co., Ltd.), which is a nonionic surfactant and also an organic solvent-based surfactant, or MIXPOWER (registered trademark; 40.0% of polyoxyethylenealkyl ether, 40.0% of polyoxyethylene alkyl phenyl ether; manufactured by Syngenta Global AG) was added to, and mixed with, an aqueous phage solution including the 1st phage (Myoviridae), the 2nd phage (Autographiviridae), or the 3rd phage (Myoviridae) to prepare the respective phage compositions. In the preparation of the compositions, the concentration of each of DRIVER and MIXPOWER was adjusted so as to be 3000-fold diluted. Preparations were made in such a manner that the titer measured immediately after preparation was the same degree of titer (10⁴ PFU/mL) in the same manner as in Example above.

Each phage composition prepared was left to stand in a thermostatic chamber at 25°C overnight (approximately 16 hours). Then, using the plaque assay method, the titer of each phage composition was calculated. The ratio of the titer of the composition having a sticking agent added therein relative to the titer of the composition having no sticking agent added therein was calculated.

### Results

The ratio of titer of the phage composition having Silwet L-77 added therein to the phage composition having no sticking agent added therein was approximately 1.0. With Silwet L-77 added, almost no decrease in titer was observed in any of the phages. On the other hand, in Comparative Example, the ratio of titer of the phage composition having DRIVER or MIXPOWER added therein to the phage composition having no sticking agent added therein was approximately 0.6 to 0.8. Thus, a decrease in titer was observed in the phage composition having DRIVER or MIXPOWER mixed therein. The above-described results have verified that the bacteriolytic activity of the phage may remarkably decrease in some cases, depending on the kind of the sticking agent mixed in, but that the organic silicone-based surfactant caused the phage to undergo a smaller decrease in the bacteriolytic activity or no decrease in the bacteriolytic activity.

### <Example 2 and Comparative Example 2>

### (Aim)

The residual amount of a bacteriophage after application of spray solution of phage containing an organic silicone-based surfactant on a peach leaf is evaluated.

### (Method and Results)

### Dispersing of solution of mixture of phage and sticking agent on excised peach leaf

Silwet L-77 (manufactured by Momentive Performance Materials, Inc.) as an organic silicone-based surfactant was added to, and mixed with, an aqueous phage solution containing the 1st phage (Myoviridae) in water to prepare a phage composition (also referred to as a spray solution of the 1st phage), which, as a composition for plant disease control, was dispersed on an excised peach leaf. Then, the residual amount of the phage was evaluated (Example 2). The amount of addition of the organic silicone-based surfactant was adjusted so as to be a 3000-fold diluted concentration. The phage concentration of the spray solution of phage prepared is commonly expressed using the titer [PFU/mL] based on the plaque forming unit (PFU) in a plaque assay method. The spray solution of phage, as a composition for plant disease control, was prepared so as to be 10⁶ PFU/mL.

Additionally, an aqueous phage solution (10⁶ PFU/mL) having no organic silicone-based surfactant added therein was prepared as a control, and evaluated as a composition for plant disease control in the same manner (Comparative Example 2).

In such a manner that the spray solution of phage prepared spreads sufficiently on the excised peach leaf, the solution was dispersed at approximately 2.5 mL per leaf. The solution was dispersed on 3 leaves per condition (n = 3). Immediately after the dispersion, a small circular piece (day 0 sample) having a diameter of 1 cm was cut out at 3 sites from the excised peach leaf. Then, the excised peach leaf was inserted in a corning tube containing absorbent cotton wetted to prevent drying. The rack was turned sideways and the tube was left to stand in an incubator at 25°C. The leaf was left to stand in the incubator for 4 days. Then, a small circular piece (day 4 sample) was cut out at 3 sites.

Each one of the small circular piece samples cut out (the day 0 sample and the day 4 sample) was inserted into a tube, to which 0.1 mL of sterile water was added. The resulting mixture was stirred and centrifuged to yield a supernatant, which was then collected as a leaf-surface extract solution of the excised peach leaf.

Then, 0.1 mL of sterile water was again added to the tube containing the excised peach leaf sample from which the leaf-surface extract solution had been obtained. The small circular piece was crushed by a beads-shocker. A supernatant obtained after centrifugation was collected as an internal extract solution of the excised peach leaf.

### Evaluation of residual amount of phage by qPCR

On the day 0 samples immediately after the dispersion and the day 4 samples after an elapse of 4 days, qPCR was performed to quantify the phage for measurement of the residual amounts of the phages from the resulting leaf-surface extract solutions and internal extract solutions. Specifically, a calibration curve was prepared using a solution the titer of phage of which had been adjusted. On the basis of the calibration curve, the titer was calculated from the Ct value of the sample solution, and the phage remaining in the peach leaf was quantified. The extract solution of the excised peach leaf, prepared in the previous section, was diluted 10-fold with sterile water, and used as a template for qPCR.

Using Powerup SYBR Green Master Mix (Applied Biosystems) as a qPCR enzyme, a reaction solution was prepared in accordance with the method described in the manual. The PCR conditions were set as follows: 50°C for 2 min.; 95°C for 2 min.; and 40 cycles of 95°C for 15 sec, 58°C for 15 sec and 72°C for 1 min. In the Table below, a primer set used is shown. No primer dimer was observed, and the PCR reaction progressed normally. As the result of qPCR, a Ct value under each condition was obtained.

**[Table 1]**

| Primer | Sequence (5' → 3') | Length (bp) |
|---|---|---|
| SEQ ID NO: 1 | TTTCTGAGTTCTCGCCCCAC | 130 |
| SEQ ID NO: 2 | GCGGACGTATTTCCGCTTTC | |

To calculate, from the Ct value obtained, the concentrations of the phage present on the surface of the excised peach leaf and inside of the leaf, it is necessary to prepare a calibration straight line between the titer [PFU/mL] in the plaque assay method and the Ct value. To perform the plaque assay method, the bacterium shown in the Table below was used as a host. Bacterium of *Xanthomonas* genus was used as a plant pathogenic bacterium, and the bacterial strain was obtained from the National Agriculture and Food Research Organization (NARO).

**[Table 2]**

| ID | Name of species | Source of separation | Place of picking |
|---|---|---|---|
| MAFF No. 311351 | *Xanthomonas arboricola* pv. *Pruni* | Peach | FUKUSHIMA |

To culture the above-described bacterium, 1 g of peptone, 1 g of a yeast extract, and 2 g of glucose were dissolved in 0.2 L of water, and autoclaved to be sterilized. The resulting liquid culture medium (YPG Broth) was used. Additionally, agar was added at 15 g per L to the above-described Broth (YPG Broth) and autoclaved to be sterilized, and the resulting agar culture medium (referred to as "YPG Agar") was used. Furthermore, to prepare a soft agar culture medium (Top Agar) to be superposed on the upper layer of the agar culture medium, agarose was added at 5 g per L to the above-described Broth (YPG Broth), and autoclaved to be sterilized. The resulting Top Agar was stored at approximately 50°C, and utilized when necessary.

For the purpose of calculating the titer in accordance with the plaque assay method, the bacterial suspension and the phage-containing solution were mixed in equal amounts, and left at room temperature for approximately 10 minutes. Next, 0.2 mL of the bacteria/phage mixture liquid was added to 3 mL of Top Agar, quickly mixed with a vortex mixer, and then poured on YPG Agar. After the Top Agar was solidified, static culture was performed at 25°C for approximately 12 hours. On the bacterial lawn formed by the culture, a bacteriolytic plaque was formed. The plaque forming unit was measured to calculate the titer.

Next, to prepare a calibration curve, a phage solution the titer of which was adjusted to 10⁶ to 10² PFU/mL was prepared. From the Ct value obtained in qPCR, a calibration curve between the titer and the Ct value was prepared. Using this calibration curve, the titer of the extract solution of the excised peach leaf was determined. The template solution used was a liquid obtained by diluting 0.1 mL of the extract solution 10-fold, and thus, this has the same meaning as a diluting operation with 1 mL. Thus, the unit of the titer of the leaf extract solution obtained is PFU. To make a conversion from the titer obtained to the residual amount of the phage per unit area on the excised peach leaf, the area of the small circular piece was calculated at 0.5, × 0.5 × 3.14 = 0.785 cm², and the residual amount of the phage per unit area (PFU/cm²) was calculated.

### Results

The results are shown in Figure 1. Figure 1 relates to a test example (Comparative Example 2) having no organic silicone-based surfactant added therein and a test example (Example 2) having an organic silicone-based surfactant added therein. Figure 1A graphs the residual amount (PFU/cm²) of a phage on the leaf surface of the day 0 sample; Figure 1B graphs the residual amount (PFU/cm²) of a phage on the leaf surface of the day 4 sample; Figure 1C graphs the residual amount (PFU/cm²) of a phage inside the leaf of the day 0 sample; and Figure 1D graphs the residual amount (PFU/cm²) of a phage inside the leaf of the day 4 sample.

As shown in Figure 1, the spray solution of phage (Example 2) having an organic silicone-based surfactant added therein, compared with the solution having no such addition (Comparative Example 2), allowed the residual amount of the phage after an elapse of 4 days to increase both on the surface of the excised peach leaf and inside the leaf. This indicates that the organic silicone-based surfactant has the effect of increasing the residual amount of the phage in application of the phage to a leaf.

### <Example 3 and Comparative Example 3>

### (Aim)

The effect of an organic silicone-based surfactant on enhancement of the residual amount of the phage is evaluated in a system in which a plurality of species of phages (the 4th phage (Siphoviridae) and the 5th phage (Siphoviridae)) different in the genome from Example 2 were used. As an organic silicone-based surfactant, MAKUPIKA (manufactured by Ishihara Sangyo Kaisha, Ltd.) was also used in addition to SL used in Example 2.

### (Method and Results)

### Dispersion of solution of mixture of phage and sticking agent on excised peach leaf

A spray solution of the 4th phage (10⁶ PFU/mL) and a spray solution of the 5th phage (10⁶ PFU/mL) as compositions for plant disease control were prepared by the same method as in Example 2 except that the 4th phage or the 5th phage was used in place of the 1st phage. Additionally, a spray solution of the 4th phage (10⁶ PFU/mL) and a spray solution of the 5th phage (10⁶ PFU/mL) that both had, as an organic silicone-based surfactant, MAKUPIKA added therein in place of Silwet L-77 (SL) were prepared. The amount of addition of the organic silicone-based surfactant was adjusted so as to be a 3000-fold diluted concentration. That is, in the present test example, the spray solution of the 4th phage (Example 3-1) having an organic silicone-based surfactant (SL) added therein, the spray solution of the 4th phage (Example 3-2) having an organic silicone-based surfactant (MAKUPIKA) added therein, the spray solution of the 4th phage (Comparative Example 3-1) having no organic silicone-based surfactant added therein, the spray solution of the 5th phage (Example 3-3) having an organic silicone-based surfactant (SL) added therein, the spray solution of the 5th phage (Example 3-4) having an organic silicone-based surfactant (MAKUPIKA) added therein, and the spray solution of the 5th phage (Comparative Example 3-2) having no organic silicone-based surfactant added therein were each prepared, and dispersed on the excised peach leaf. The residual amount of the phage was evaluated. On the excised peach leaf, each spray solution in an amount of approximately 2.5 mL per leaf was dispersed. Under each condition, 3 leaves were used (n = 3). From the excised peach leaf, after dispersion of the spray solution of phage, a leaf-surface extract solution and an internal extract solution were prepared by the same method as in Example 2.

### Evaluation of residual amount of bacteriophage by qPCR

qPCR was performed by the same method as described in Example 2 except that the primers listed in the Table below were used. Using the day 0 sample immediately after the dispersion and the day 4 sample after an elapse of 4 days, the residual amounts of the phage were measured from the resulting leaf-surface extract solution and internal extract solution.

**[Table 3]**

| Primer | Sequence (5' → 3') | Length (bp) |
|---|---|---|
| SEQ ID NO: 3 | TTTTTCTCGTCTGAGGCAGG | 115 |
| SEQ ID NO: 4 | GTGCTATGGTTCGGGACTTT | |
| SEQ ID NO: 5 | TCTTCGTCTCGGGCTTCTT | 102 |
| SEQ ID NO: 6 | CCGTTCTCCTTGTTGATGGT | |

### Results

The results are shown in Figures 2 and 3. Figure 2 relates to test examples including use of a 4th phage, as follows: a test example (Comparative Example 3-1) having no organic silicone-based surfactant added therein; a test example (Example 3-1) having an organic silicone-based surfactant (SL) added therein; or a test example (Example 3-2) having an organic silicone-based surfactant (MAKUPIKA) added therein. Figure 2A graphs the residual amount (PFU/cm²) of a phage on the leaf surface of the day 0 sample; Figure 2B graphs the residual amount (PFU/cm²) of a phage on the leaf surface of the day 4 sample; Figure 2C graphs the residual amount (PFU/cm²) of a phage inside the leaf of the day 0 sample; and Figure 2D graphs the residual amount (PFU/cm²) of a phage inside the leaf of the day 4 sample. Additionally, Figure 3 relates to test examples including use of a 5th phage, as follows: a test example (Comparative Example 3-2) having no organic silicone-based surfactant added therein, a test example (Example 3-3) having an organic silicone-based surfactant (SL) added therein, or a test example (Example 3-4) having an organic silicone-based surfactant (MAKUPIKA) added therein. Figure 3A graphs the residual amount (PFU/cm²) of a phage on the leaf surface of the day 0 sample; Figure 3B graphs the residual amount (PFU/cm²) of a phage on the leaf surface of the day 4 sample; Figure 3C graphs the residual amount (PFU/cm²) of a phage inside the leaf of the day 0 sample; and Figure 3D graphs the residual amount (PFU/cm²) of a phage inside the leaf of the day 4 sample.

The results of Figures 2 and 3, put together, are shown in the Table below.

**[Table 4]**

| | Phage | Surfactant | Residual amount of phage (PFU/cm²) | | | | | |
|---|---|---|---|---|---|---|---|---|
| | | | Immediately after application | | | After elapse of 4 days | | |
| | | | Leaf surface | Leaf inside | Total | Leaf surface | Leaf inside | Total |
| Comparative example 3-1 | 4th | - | 5080 | 365 | 5445 | 68 | 33 | 101 |
| Example 3-1 | 4th | SL | 4209 | 459 | 4668 | 659 | 175 | 834 |
| Example 3-2 | 4th | MAKUPIKA | 2514 | 342 | 2856 | 303 | 102 | 405 |
| Comparative example 3-2 | 5th | - | 5382 | 330 | 5712 | 455 | 38 | 493 |
| Example 3-3 | 5th | SL | 5210 | 385 | 5595 | 665 | 290 | 955 |
| Example 3-4 | 5th | MAKUPIKA | 3024 | 657 | 3681 | 278 | 489 | 767 |

As shown in Figures 2 and 3 and Table 4, the spray solution of the 4th phage or the spray solution of the 5th phage (Example 3-1 or Example 3-3) having an organic silicone-based surfactant (SL) added therein, compared with the spray solution of phage having no such addition (Comparative Example 3-1 or Comparative Example 3-2), allowed the residual amount of the phage after an elapse of 4 days to increase both on the surface of the excised peach leaf and inside the leaf. Additionally, the spray solution of the 4th phage or the spray solution of the 5th phage (Example 3-2 or Example 3-4) having an organic silicone-based surfactant (MAKUPIKA) added therein, compared with the spray solution of phage having no such addition (Comparative Example 3-1 or Comparative Example 3-2), allowed the residual amount of the phage after an elapse of 4 days to increase at least in the total amount of the phage on the leaf surface and the phage inside the leaf. These results have revealed that the addition of an organic silicone-based surfactant increased the residual amount of the phage, independent of the species of the phage and the kind of the organic silicone-based surfactant.

### <Example 4 and Comparative Example 4>

### (Aim)

The residual amount of a bacteriophage after dispersion of a phage solution containing an organic silicone-based surfactant on an excised broccoli leaf or an excised tomato leaf is evaluated.

### (Method and Results)

### Dispersion of solution of mixture of phage and sticking agent on excised broccoli leaf or excised tomato leaf

Using the same method as in Example 2, Silwet L-77 (SL) as an organic silicone-based surfactant was added to an aqueous phage solution containing the 1st phage (Myoviridae) to prepare a spray solution of phage (10⁶ PFU/mL), which was then dispersed on an excised broccoli leaf or an excised tomato leaf. The residual amount of the phage was evaluated (Examples 4-1 and 4-2). The amount of addition of the organic silicone-based surfactant was adjusted so as to be a 3000-fold diluted concentration. The spray solution of phage prepared was dispersed at approximately 2.0 mL per leaf on the excised tomato leaf and at approximately 5.0 mL per leaf on the excised broccoli leaf in such a manner that the solution spreads sufficiently on the excised leaf. In this regard, the solution was dispersed on 3 leaves per condition (n = 3). From each of the excised broccoli leaves and the excised tomato leaves, after dispersion of the spray solution of phage, the leaf-surface extract solution and the internal extract solution were prepared by the same method as in Example 2.

Additionally, an aqueous phage solution (10⁶ PFU/mL) having no organic silicone-based surfactant added therein was prepared as a control example, and evaluated in the same manner (Comparative Examples 4-1 and 4-2).

### Evaluation of residual amount of bacteriophage by qPCR

Using the same method as described in Example 2, the excised broccoli leaves and the excised tomato leaves were treated to prepare extract solutions (leaf-surface extract solutions and internal extract solutions). Using the same method as in Example 2, qPCR was performed on the extract solutions, and the residual amount of the bacteriophage was evaluated. As the primers for qPCR, the primers shown in Table 1 were used.

### Results

The results are shown in Figures 4 and 5. Figure 4 relates to test examples including use of an excised broccoli leaf, as follows: a test example (Comparative Example 4-1) having no organic silicone-based surfactant added therein and a test example (Example 4-1) having an organic silicone-based surfactant (SL) added therein. Figure 4A graphs the residual amount (PFU/cm²) of a phage on the leaf surface of the day 0 sample; Figure 4B graphs the residual amount (PFU/cm²) of a phage on the leaf surface of the day 4 sample; Figure 4C graphs the residual amount (PFU/cm²) of a phage inside the leaf of the day 0 sample; and Figure 4D graphs the residual amount (PFU/cm²) of a phage inside the leaf of the day 4 sample. Additionally, Figure 5 relates to test examples including use of an excised tomato leaf, as follows: a test example (Comparative Example 4-2) having no organic silicone-based surfactant added therein and a test example (Example 4-2) having an organic silicone-based surfactant (SL) added therein. Figure 5A graphs the residual amount (PFU/cm²) of a phage on the leaf surface of the day 0 sample; Figure 5B graphs the residual amount (PFU/cm²) of a phage on the leaf surface of the day 4 sample; Figure 5C graphs the residual amount (PFU/cm²) of a phage inside the leaf of the day 0 sample; and Figure 5D graphs the residual amount (PFU/cm²) of a phage inside the leaf of the day 4 sample.

The results of Figures 4 and 5 are shown in the Table below.

**[Table 5]**

| | Excised leaf | Surfactant | Residual amount of phage (PFU/cm²) | | | | | |
|---|---|---|---|---|---|---|---|---|
| | | | Immediately after dispersion | | | After elapse of 4 days | | |
| | | | Leaf surface | Leaf inside | Total | Leaf surface | Leaf inside | Total |
| Comparative example 4-1 | Broccoli | - | 6926 | 4423 | 11349 | 4340 | 2542 | 6882 |
| Example 4-1 | Broccoli | SL | 14286 | 7483 | 21769 | 11754 | 5314 | 17068 |
| Comparative example 4-2 | Tomato | - | 19205 | 2005 | 21210 | 2904 | 1009 | 3913 |
| Example 4-2 | Tomato | SL | 18637 | 6426 | 25063 | 10078 | 4142 | 14220 |

As shown in Figures 4 and 5 and Table 5, the spray solution of phage (Example 4-1 or 4-2) having an organic silicone-based surfactant (SL) added therein, compared with the spray solution of phage having no such addition (Comparative Example 4-1 or 4-2), allowed the residual amount of the phage after an elapse of 4 days to increase both on the surface of and inside the excised broccoli leaf and the excised tomato leaf in the same manner as in the excised peach leaf. This indicates that, also for a plant leaf other than the excised peach leaf, the addition of an organic silicone-based surfactant can enhance the residual amount of the phage.

### <Example 5 and Comparative Example 5>

### Aim)

The effect of an organic silicone-based surfactant on enhancement of the residual amount of the phage is evaluated in a system in which a plurality of species of phages (the 2nd phage (Autographiviridae) and the 3rd phage (Myoviridae) used in Example 1) different in the genome from Examples 2 and 3 were used. As an organic silicone-based surfactant, SL used in Example 2 was used.

### (Method and Results)

### Dispersion of solution of mixture of phage and sticking agent on excised peach leaf

A spray solution of the 2nd phage (10⁶ PFU/mL) and a spray solution of the 3rd phage (10⁶ PFU/mL) as compositions for plant disease control were prepared by the same method as in Example 2 except that the 2nd phage or the 3rd phage were used in place of the 1st phage. The amount of addition of the organic silicone-based surfactant was adjusted so as to be a 3000-fold diluted concentration. That is, in the present test example, the spray solution of the 2nd phage having an organic silicone-based surfactant (SL) added therein, the spray solution of the 2nd phage having no organic silicone-based surfactant added therein, the spray solution of the 3rd phage having an organic silicone-based surfactant (SL) added therein, and the spray solution of the 3rd phage having no organic silicone-based surfactant added therein were each prepared, and dispersed on the excised peach leaf. The residual amount of the phage was evaluated. On the excised peach leaf, each solution in an amount of approximately 2.5 mL per leaf was dispersed. Under each condition, 3 leaves were used (n = 3).

### Evaluation of residual amount of bacteriophage by qPCR

qPCR was performed by the same method as described in Example 2 except that the primers listed in the Table below were used. Using the day 4 sample after an elapse of 4 days, the residual amounts of the phage were measured from the resulting leaf-surface extract solution and internal extract solution.

**[Table 6]**

| Primer | Sequence (5' → 3') | Length (bp) |
|---|---|---|
| SEQ ID NO: 7 | GAACTTTCCACGGTTGGTGC | 132 |
| SEQ ID NO: 8 | CGAACGGATTACGAGCGAGA | |
| SEQ ID NO: 9 | CGCCAATAGCTCGGGATTCT | 135 |
| SEQ ID NO: 10 | CAATACGCAGAAAGGCGACG | |

### Results

The condition having the 2nd phage and SL, and 3rd phage and SL added therein, compared with condition having no such addition, allowed the residual amount of the phage after an elapse of 4 days to increase. This indicates that the addition of an organic silicone-based surfactant increases the residual amount of the phage in a plant leaf, independent of the species of the phage.

### <Example 6 and Comparative Example 6>

### Bacterial strain used

Tomato: *Xanthomonas campestris* pv. *vesicatoria* (MAFF No. 301256)
Broccoli: *Xanthomonas campestris* pv. *campestris* (MAFF No. 106765)

### Phages used (cocktails of 4 kinds of phages)

Tomato: 4th phage (Siphoviridae), 6th phage (Myovirdae), 5th phage (Siphoviridae), and 7th phage (Siphoviridae)
Broccoli: 4th phage (Siphoviridae), 6th phage (Myovirdae), 8th phage (Autographiviridae), and 7th phage (Siphoviridae)

### Test for plant disease control effect

### (1) Preparation of sample

A spray solution of phage used in this test was prepared in accordance with the following procedure. First, the bacterial strain was inoculated into a YPG culture liquid, and cultured overnight in a shaker set at 25°C to prepare a bacterial cell culture liquid having an OD₆₀₀ (turbidity at 600 nm) of approximately 1.0. Then, this bacterial cell culture liquid and a refined solution of phage (having a titer of approximately 10⁸ PFU/mL) prepared separately were mixed in equal amounts. The resulting mixture liquid was inoculated into a 100-fold amount of YPG culture liquid, and cocultured for approximately 8 to 12 hours in a shaker set at 25°C. The resulting culture liquid was collected. When the titer after the culture was insufficient, the mixing ratio of the culture liquid to the refined solution of phage in the coculture was fine-tuned, and the liquid and the solution were cocultured again. To the resulting liquid collected, a 1/10 amount of chloroform was added, stirred vigorously, and then centrifuged. The supernatant was collected in such a manner that the chloroform in the lower layer was not touched. Furthermore, a phage-containing solution obtained by allowing this supernatant to pass through a 0.2 µm filter was used as a massive-phage preparation solution free from contamination of a bacterial cell. This massive-phage preparation solution was diluted with sterile tap water so as to have a titer of approximately 10⁹ PFU/mL, and adjusted so as to have an organic silicone-based surfactant SL diluted 3000-fold (Example 6). Separately, the massive phage preparation solution was diluted with sterile tap water so as to have a titer of approximately 10⁹ PFU/mL (Comparative Example 6). The resulting solutions were used as final spray solutions of phage. For the cocktails of phages, the massive-phage preparation solutions adjusted so as to have the titer of the same order were individually mixed in equal amounts, and then, resulting solution was used as a stock solution before dilution.

A spray solution of bacteria used for infection treatment of a plant in this test was prepared using several YPG plates, as follows: a bacterial cell culture liquid not (before) having the above-described refined solution of phage mixed therein was diluted approximately 10,000-fold with sterile tap water; and the resulting solution was spread to the plates, and underwent a solid culture for approximately 1 to 3 days in an incubator set at 25°C. Colonies on the solid-culture plates were collected while the colonies were suspended in sterile tap water. The resulting suspension was diluted with sterile tap water so as to have a final OD₆₀₀ (turbidity at 600 nm) of approximately 0.5. The resulting bacteria-containing solution was used as a spray solution of bacteria.

### (2) Plants

Commercially available tomato seeds and broccoli seeds were seeded, and grown in a greenhouse. A tomato seedling grown to have 50 or more leaves and a broccoli seedling grown to have 5 or more leaves were utilized as evaluation specimens. On each specimen, the spray solution of phage was dispersed on the leaf face before and after a bacterial infection treatment, twice each, at intervals of 2 to 3 days. For each spray solution, 8 specimens were prepared. Nontreated groups were as follows: in (1) above, sterile tap water was used in place of the refined solution of phage; bacteria alone were mass-cultured; then, the culture liquid underwent aseptic treatment, and was diluted with sterile tap water in the same manner; and the resulting spray solution was dispersed on the leaf face. As a result, the nontreated groups were substantially the same except containing no phage in the spray solution. In some cases, a plurality of branches of the same seedling was separated to two groups to undergo respective phage application. The bacterial infection was conducted, as follows: the spray solution of bacteria was dispersed on the leaf face; then, the seedlings were placed in a simple polytunnel the inside of which was sufficiently wetted with sterile tap water, and the polytunnel was hermetically sealed; and the seedlings were left for approximately 2 days at a humidity higher than in the surroundings. When some degree of disease development was observed in the nontreated group after an elapse of 1 week or more beginning from the infection treatment, the disease incidence rate in the case of application of each spray solution was examined. Bacterial spot observed in peaches develops a peculiar brown spot on the leaf face. Thus, the ratio of the number of leaves developing the disease observed therein to the total number of leaves at the time of examination was regarded as a disease incidence rate.

### (3) Test for control effect of each species of phage

By verifying the disease incidence rate in accordance with the above-described method, both the tomato and the broccoli were evaluated 20 days after the infection treatment. In some cases, broccoli has a smaller total number of leaves (denominator) than a tomato, has a larger leaf area, and thus, has a larger risk of not having the spray solution spread on the whole leaf. Accordingly, with respect to the values of the nontreated groups, the disease incidence rate of the broccoli can be worse than the disease incidence rate of the tomato.

### (4) Results

The results are shown in the Table below.

**[Table 7]**

| | Excised leaf | Surfactant | Disease incidence rate (%) |
|---|---|---|---|
| Comparative example 6-1 | Tomato | - | 19.7 |
| Example 6-1 | Tomato | SL | 16.9 |
| Comparative example 6-2 | Broccoli | - | 25.8 |
| Example 6-2 | Broccoli | SL | 22.2 |

As shown in Table 7, it has been verified that the spray solution of phage (Example 6-1 or 6-2) having an organic silicone-based surfactant (SL) added therein was decreased in the disease incidence rate, compared with the spray solution of phage having no such addition (Comparative Example 6-1 or 6-2). This is considered to be because the addition of an organic silicone-based surfactant enhanced the residual amount of the phage, and thus, was able to control a plant disease.

The upper limits and/or lower limits of the ranges of values herein described can be arbitrarily combined to define a preferred range. For example, any upper limit and any lower limit of the ranges of values can be combined to define a preferred range. Any upper limits of the ranges of values can be combined to define a preferred range. Any lower limits of the ranges of values can be combined to define a preferred range.

All publications, patents, and patent applications cited herein are incorporated herein by reference in their entirety.

## Claims

1. A composition for plant disease control, comprising:
a bacteriophage with bacteriolytic activity against bacteria; and
an organic silicone-based surfactant.

2. The composition for plant disease control according to claim 1, wherein the organic silicone-based surfactant is a silicone oil having a polyether group(s).

3. The composition for plant disease control according to claim 1, wherein the organic silicone-based surfactant is at least one selected from the group consisting of trisiloxaneethoxylate, polyoxyethylene methylpolysiloxane, polyoxyalkylene methylpolysiloxane, and a polyether polymethylsiloxane copolymer.

4. The composition for plant disease control according to claim 1, wherein the organic silicone-based surfactant is trisiloxaneethoxylate.

5. The composition for plant disease control according to claim 1, wherein the organic silicone-based surfactant is polyalkylene-oxide-modified heptamethyltrisiloxane.

6. The composition for plant disease control according to claim 1, further comprising an agriculturally acceptable carrier and/or an agriculturally acceptable solvent.

7. A method for controlling plant disease, comprising a step of contacting the composition for plant disease control according to claim 1 to a target plant.

8. A method for controlling plant disease, comprising:
a step of preparing a composition for plant disease control by mixing a bacteriophage with bacteriolytic activity against bacteria with an organic silicone-based surfactant; and
a step of contacting the composition for plant disease control to a target plant to be controlled.

9. The method for controlling plant disease according to claim 8, wherein the organic silicone-based surfactant is a silicone oil having a polyether group(s).

10. The method for controlling plant disease according to claim 8, wherein the organic silicone-based surfactant is at least one selected from the group consisting of trisiloxaneethoxylate, polyoxyethylene methylpolysiloxane, polyoxyalkylene methylpolysiloxane, and a polyether polymethylsiloxane copolymer.

11. The method for controlling plant disease according to claim 8, wherein the organic silicone-based surfactant is trisiloxaneethoxylate.

12. The method for controlling plant disease according to claim 8, wherein the organic silicone-based surfactant is polyalkylene-oxide-modified heptamethyltrisiloxane.

13. The method for controlling plant disease according to claim 8, wherein the step of preparing comprises preparing a composition for plant disease control by further mixing an agriculturally acceptable carrier and/or an agriculturally acceptable solvent in addition to the bacteriophage and the organic silicone-based surfactant.

14. Use of an organic silicone-based surfactant in a composition for plant disease control containing a bacteriophage with bacteriolytic activity against bacteria, wherein the use is for enhancing the residual amount of the composition for plant disease control in a target plant.

15. Use of an organic silicone-based surfactant in the production of a composition for plant disease control containing a bacteriophage with bacteriolytic activity against bacteria.
